# EUROPÄISCHE PATENTANMELDUNG

(11) **EP 2 177 901 A2**
(43) Veröffentlichungstag der Anmeldung: **21.04.2010**
(21) Anmeldenummer: 09170627.5
(22) Anmeldetag: 18.09.2009
(51) Int. Cl.: G01N 27/28, G01R 31/04, H01R 13/66

(54) **Messvorrichtung und Verfahren für eine Feuchtigkeitsdetektion an einem Messspannungseingang einer solchen Messvorrichtung**

(30) Priorität: 15.10.2008 DE 102008052813
(71) Anmelder: Endress+Hauser Conducta Gesellschaft für Mess- und Regeltechnik mbH+Co. KG, 70839 Gerlingen (DE)
(72) Erfinder: Buschnakowski, Stephan, 09127, Chemnitz (DE); Lohmann, Martin, 70839, Gerlingen (DE); Straub, Hermann, 72108, Rottenburg (DE); Uhle, Jörg, 09212, Limbach (DE)
(74) Vertreter: Andres, Angelika Maria

(57) **Zusammenfassung**

Es werden eine Messvorrichtung mit einem Messspannungseingang (1) mit zumindest einem Eingangskontakt (2) für eine Eingangsspannung (U_{E}) von einem Messelement und ein Verfahren für eine Feuchtigkeitsdetektion an dem Messspannungseingang (1) einer solchen Messvorrichtung vorgeschlagen, wobei die Messvorrichtung eine Zusatzspannungsquelle (3) mit zumindest einer Zusatzspannung (U_{Z}) aufweist, verbunden mit einem Zusatzkontakt (4), angeordnet im Bereich des zumindest einen Eingangskontakts (2).

## Beschreibung

Die Erfindung betrifft eine Messvorrichtung mit einem Messspannungseingang mit zumindest einem Eingangskontakt für eine Eingangsspannung U_{E} von einem Messelement gemäß dem Oberbegriff des Anspruchs 1 und ein Verfahren für eine Feuchtigkeitsdetektion an einem Messspannungseingang einer solchen Messvorrichtung gemäß dem Oberbegriff des Anspruchs 5.

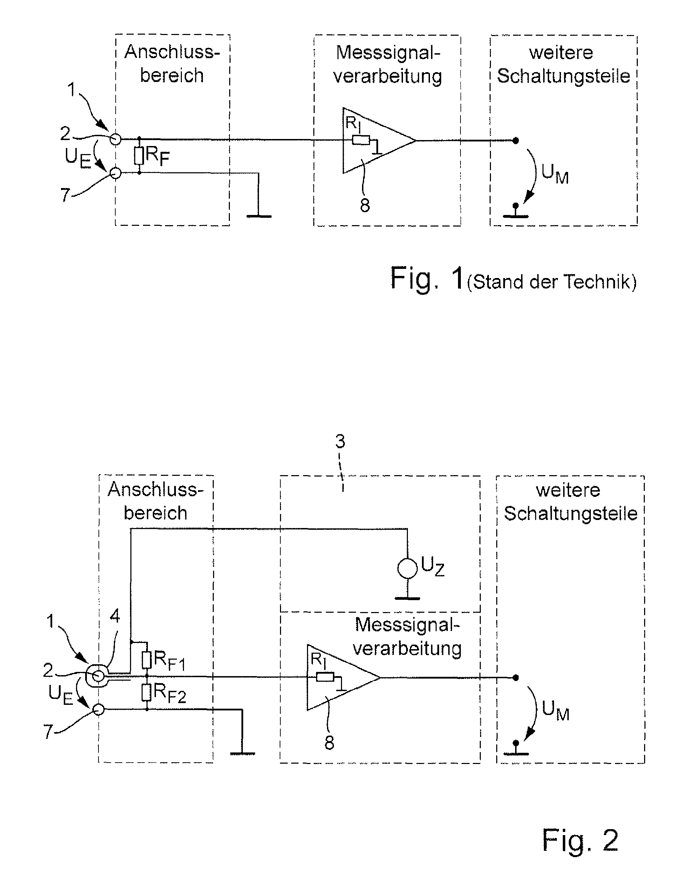

Eine Messvorrichtung des Standes der Technik zur Spannungsmessung eines Messsignals von einem Messelement, z.B. einem Sensorelement wie etwa einem Glassensor für die pH-Wert Messung, wird schematisch und vereinfacht in Fig. 1 dargestellt. Von dem Messelement, in Fig. 1 nicht dargestellt, wird das Messsignal mit einer Eingangsspannung U_{E} über einen Anschlussbereich einer Messvorrichtung über den Messspannungseingang 1 mit zumindest einem Eingangskontakt 2 und einem Referenz-Eingangskontakt 7 zugeführt. Der Anschlussbereich muss dabei zumindest den Eingangskontakt 2 aufweisen. Der Referenz-Eingangskontakt 7 ist ein Referenzpotential bzw. ist mit einem Referenzpotential (z.B. Masse) verbunden. Der Eingangskontakt 2 ist mit einem hochohmigen Eingang eines Spannungsfolgers/lmpedanzwandlers 8 verbunden. Nach dem Impedanzwandler 8 kann das Messsignal mit einer Messpannung UM niederohmig weiterverarbeitet werden, beispielsweise durch weitere Analogsignalverarbeitung, Analog-Digital-Wandlung usw. Dies wird hier nicht näher beschrieben und ist dem Fachmann geläufig. Durch die Hochohmigkeit des Impedanzwandlers 8 mit seinem hochohmigen Eingangswiderstand R_{I} können auch Spannungssignale eines Messelements mit großem Innenwiderstand gemessen werden, wie beispielsweise von Glassensoren für die pH-Wert Messung.

Es besteht jedoch bei solchen Messvorrichtungen des Standes der Technik der Nachteil, dass der hochohmige Messspannungseingang 1 empfindlich gegen elektrische Beeinflussung ist. So sind zum Beispiel Leckwiderstandspfade kritisch, die den hochohmigen Messspannungseingang 1 kontaktieren. Liegt ein ohmscher Leckwiderstand, in Fig. 1 durch R_{F} schematisch dargestellt, beispielsweise im Bereich der Größe des Innenwiderstands des verwendeten Messelements oder kommt er dem Bereich der Größe dieses Innenwiderstands des Messelements nahe, tritt eine Verfälschung des Signals des Messelements auf. Wie in Fig. 1 schematisch gezeigt, kann der Leckwiderstand R_{F} als Parallelwiderstand zum Eingangswiderstand der Messvorrichtung betrachtet werden.

Eine Ursache für die Ausbildung des Leckwiderstands R_{F} am hochohmigen Messspannungseingang 1 kann zum Beispiel die Anlagerung von Feuchtigkeit sein. Bei Einsatz einer elektronischen Messvorrichtung in der Umgebung von flüssigen Medien besteht im Fehlerfall die Möglichkeit einer solchen Feuchtigkeitsansammlung. Dies kann etwa bei Anwendungen auftreten, bei welchen eine solche elektronische Messvorrichtung als Sensor-vor-Ort-Elektronik in der Flüssigkeitsanalyse eingesetzt wird, d. h. eine solche Messvorrichtung wird direkt und in örtlicher Nähe mit einem Messelement verbunden, welches selbst flüssige Medien beinhaltet und/oder in flüssigen Medien betrieben wird.

Ist nun zum Beispiel bei einer solchen Messvorrichtung die Abdichtung zum flüssigen Medium beispielsweise durch fehlerhaften mechanischen Aufbau oder mechanische Belastung fehlerhaft, kann Feuchtigkeit an die elektronische Messvorrichtung, insbesondere an deren Messspannungseingang 1, gelangen. Dies führt dann zu einer Verfälschung des Messergebnisses. So bilden in der Praxis solche Feuchtigkeitsanlagerungen zuerst Leckpfade mit dem Leckwiderstand R_{F} im Bereich des Eingangskontakts 2 und des ReferenzEingangskontakts 7 des Messspannungseingangs 1 aus. Da in der Regel der Referenz-Eingangskontakt 7 niederohmig an das Referenzpotential (z. B. Masse) angebunden ist, wird der hochohmige Eingangskontakt 2 über den Leckwiderstand R_{F} ebenfalls auf dieses Referenzpotential gezogen. Handelt es sich bei dem Referenzpotential um ein mögliches Messpotential, so kann aufgrund der dabei gemessenen Spannung nicht mehr unterschieden werden, ob eine Feuchtigkeitsanlagerung am Messspannungseingang 1 der Messvorrichtung vorliegt, mithin also eine fehlerhafte Störung, oder ob die Messvorrichtung ordnungsgemäß betrieben werden kann, wobei ordnungsgemäß von dem Messelement z.B. eine Eingangsspannung U_{E} von der Größe im Bereich des Referenzpotentials an den Messspannungseingang 1 gegeben ist. Im Ergebnis beeinflusst also ein möglicher Leckwiderstand R_{F} direkt die Messgröße von einem Messelement. Es ist daher in der Praxis wünschenswert, eine Möglichkeit zu finden, sicher und zuverlässig feststellen zu können, ob eine mögliche Fehlfunktion aufgrund eines auftretenden Leckwiderstands an einem Messspannungseingang 1 in einem Anschlussbereich einer Messvorrichtung vorliegt oder ob die Messung einen unverfälschten Messwert liefert.

Daher ist es die Aufgabe der vorliegenden Erfindung, eine Messvorrichtung sowie ein Verfahren für eine Feuchtigkeitsdetektion an einem Messspannungseingang einer solchen Messvorrichtung bereitzustellen, welche auf möglichst einfache und zuverlässige Art und Weise eine Feuchtigkeitsdetektion ermöglichen.

Diese Aufgabe wird mit einer Messvorrichtung mit den Merkmalen des Anspruchs 1 sowie mit einem Verfahren für eine Feuchtigkeitsdetektion an einem Messspannungseingang einer solchen Messvorrichtung mit den Schritten des Anspruchs 5 gelöst. Vorteilhafte Ausgestaltungen und Weiterbildungen der Erfindung sind Gegenstand der jeweils abhängigen Ansprüche

Die Messvorrichtung nach der Erfindung weist einem Messspannungseingang mit zumindest einem Eingangskontakt für eine Eingangsspannung U_{E} von einem Messelement und eine Zusatzspannungsquelle auf, welche zumindest eine Zusatzspannung U_{Z} liefert, verbunden mit einem Zusatzkontakt, angeordnet im Bereich des zumindest einen Eingangskontakts.

Erfindungsgemäß wird also eine Zusatzspannungsquelle vorgesehen, welche mit einem Zusatzkontakt ein weiteres Zusatznetz bildet, welches ohne Vorhandensein von Feuchtigkeit eigentlich nicht mit dem zumindest einen Eingangskontakt des Messspannungseingangs in leitender Verbindung steht. Erfindungsgemäß ist jedoch der Zusatzkontakt nahe im Bereich des zumindest einen (hochohmigen) Eingangskontakts angeordnet, d. h. in einem minimalen Abstand um den hochohmigen Eingangskontakt eines Anschlussbereichs der Messvorrichtung herum gezogen. Die Zusatzspannungsquelle liefert zumindest eine Zusatzspannung U_{Z} an den Zusatzkontakt. Kommt es nun beispielsweise durch auftretende Feuchtigkeit im Bereich des Messspannungseingangs der erfindungsgemäßen Messvorrichtung zum Auftreten von Leckwiderständen, so wird ein erster Leckwiderstand R_{F1} zwischen dem Eingangskontakt und dem erfindungsgemäßen Zusatzkontakt auftreten, wobei durch die Eigenschaft der vorhandenen Zusatzspannung U_{Z} von der Zusatzspannungsquelle eine Detektion dieses Auftretens von Feuchtigkeit möglich ist, da der hochohmige Eingangskontakt nicht auf das Referenzpotential absinken wird, wie dies im Fall des Standes der Technik gegeben ist, sondern eher auf das Potential der angelegten Zusatzspannung U_{Z}, wobei bei Kenntnis der Eigenschaften dieser Zusatzspannung eine sichere Identifizierung dieses unerwünschten Eindringens von Feuchtigkeit und somit der potentiell verfälschten Messmöglichkeit erfindungsgemäß möglich ist. Dazu dient der relativ einfache erfindungsgemäße Aufbau. Bevorzugt handelt es sich bei der Zusatzspannung U_{Z} um eine Festspannung. Diese kann zum Beispiel eine positive oder negative Versorgungsspannung sein.

Es kann also erfindungsgemäß mit geringem Schaltungsaufwand (lediglich eine Zusatzspannungsquelle mit zumindest einer Zusatzspannung U_{Z} und ein Zusatzkontakt, angeordnet im Bereich des zumindest einen Eingangskontakts, sind nötig) eine einfache Erfassung des Eindringens von Feuchtigkeit in den Bereich des Messspannungseingangs einer Messvorrichtung erfindungsgemäß erfolgen. Es ist auch keine weitere Änderung der nachfolgenden Messwertverarbeitung notwendig. Die erfindungsgemäße Gestaltung ist somit auch kompatibel mit bestehenden Schaltungen von bestehenden Messvorrichtungen. Eine Fehlfunktion der Messschaltung aufgrund auftretender Feuchtigkeit kann somit erfindungsgemäß leicht nachgewiesen werden.

Bevorzugt kann bei der erfindungsgemäßen Messvorrichtung die Zusatzspannungsquelle so vorgesehen sein, dass sie zumindest eine Zusatzspannung U_{Z} einer Größe liefert, welche außerhalb eines Bereichs einer Größe der Eingangsspannung U_{E} liegt. Die Bedingung ist bevorzugt also nur, dass die Zusatzspannungsquelle erfindungsgemäß eine Zusatzspannung U_{Z} liefert, welche außerhalb des Bereichs der Größe der Eingangsspannung U_{E} liegt. Findet dann ein Eindringen von Feuchtigkeit in den Bereich der Kontakte des Messspannungseingangs statt, dann wird der hochohmige Eingangskontakt nicht wie beim Stand der Technik auf das Potential des Referenzeingangskontakts gezogen, sondern er wird bevorzugt nach außerhalb des Bereichs der Werte der Eingangsspannung U_{E} in Richtung des Potentials der Zusatzspannung U_{Z} des Zusatznetzes gezogen. Die Detektion von Feuchtigkeit kann somit durch Auswertung des Messsignals erfolgen. Verlässt das Messsignal den normalen Messbereich (in Richtung der Größe der Zusatzspannung U_{Z}), so ist dies ein Indiz für das Eindringen von Feuchtigkeit. Demgegenüber war es beim Stand der Technik bislang so, dass bei Eindringen von Feuchtigkeit das Messsignal, wie gesagt, auf das Potential des ReferenzEingangskontakts gezogen wurde, es fand also kein Herauslaufen des Messsignals aus dem normalen Messbereich statt.

Gemäß einer weiteren bevorzugten Ausführungsform der erfindungsgemäßen Messvorrichtung kann die Zusatzspannungsquelle so vorgesehen sein, dass sie zumindest eine erste Zusatzspannung U_{Z1} und eine zweite Zusatzspannung U_{Z2} liefert, z.B. mit einer ersten Zusatzspannungsquelle und einer zweiten Zusatzspannungsquelle, und eine Zusatzspannungsumschalteinrichtung aufweist, mit welcher zwischen der ersten Zusatzspannung U_{Z1} und der zweiten Zusatzspannung U_{Z2} umgeschaltet werden kann. Bevorzugt kann die zweite Zusatzspannung U_{Z2} dabei ein positives Spannungspotential V_{CC} sein. Die erste Zusatzspannung U_{Z1} kann dabei bevorzugt ein Referenzpatential (z.B. Masse) sein. Das erfindungsgemäße Zusatznetz kann also gemäß dieser bevorzugten Ausführungsform wechselweise zwischen zwei definierten Potentialen umschaltbar sein. Dringt nun Feuchtigkeit in den Anschlussbereich des Messspannungseingangs der erfindungsgemäßen Messvorrichtung mit dieser Gestaltung ein, wird diese Feuchtigkeit wie auch im Fall der vorherig beschriebenen Ausführungsform der erfindungsgemäßen Messvorrichtung mit hoher Wahrscheinlichkeit zuerst Kontakt mit dem Zusatznetz aus Zusatzkontakt und Zusatzspannungsquelle bekommen. Dadurch werden das Zusatznetz und der hochohmige Eingangskontakt über einen ersten Leckwiderstand R_{F1} in Verbindung gelangen. Bei entsprechend hoher Leitfähigkeit des entsprechenden Feuchtigkeitsfilms wird dies dazu führen, dass das Eingangspotential in Richtung des Potentials der Zusatzspannung U_{Z} des Zusatznetzes gezogen wird. Eine besonders sichere Detektion der auftretenden Feuchtigkeit kann dann mit Hilfe der Zusatzspannungsumschalteinrichtung ermöglicht sein. Der Spannungsverlauf der Eingangsspannung U_{E} und/oder der Messspannung UM in Abhängigkeit der Schaltvorgänge bei Umschaltung von der ersten Zusatzspannung U_{Z1} auf die zweite Zusatzspannung U_{Z2} und zurück wird beim Vorliegen eines Feuchtigkeitseinbruchs der Richtung der Spannungsänderung zwischen den beiden Zusatzspannungen folgen.

Tabelle 1 zeigt die entsprechenden Umschaltvorgänge der Zusatzspannungsumschalteinrichtung unter der Annahme, dass U_{Z1} < U_{Z2}.

| **Schritt** | **Umschaltung** | **Feuchtigkeit real vorhanden** | **U_{M}** | **Feuchtigkeit detektiert** |
|---|---|---|---|---|
| 1 | U_{Z1} → U_{Z2} | Ja | ↑ | Ja |
| 1 | U_{Z1} → U_{Z2} | Nein | - | Nein |
| 2 | U_{Z2} → U_{Z1} | Ja | ↓ | Ja |
| 2 | U_{Z2} → U_{Z1} | Nein | - | Nein |

Nur wenn also gemäß der obigen Tabelle 1 in Schritt 1 und in Schritt 2 die Messspannung UM nach dem Umschalten die gleiche Änderungsrichtung annimmt wie die Spannungsänderung von der ersten Zusatzspannung U_{Z1} zur zweiten Zusatzspannung U_{Z2} bzw. entsprechend zurück, liegt eine elektrische Verbindung über den ersten Leckwiderstand R_{F1} zum Beispiel aufgrund von Feuchtigkeit zwischen dem Zusatznetz und dem hochohmigen Eingangskontakt des Messspannungseingangs der erfindungsgemäßen Messvorrichtung vor.

Auch gemäß dieser Ausführungsform der erfindungsgemäßen Messvorrichtung kann auf einfache Weise eine sichere und zuverlässige Detektion von auftretender Feuchtigkeit erfolgen, wobei hier die Zuverlässigkeit der Detektion gegenüber der ersten bevorzugten Ausführungsform durch die Zusatzspannungsumschaltmöglichkeit nochmals weiter erhöht ist.

Gemäß einer weiteren bevorzugten Ausführungsform der erfindungsgemäßen Messvorrichtung kann die Zusatzspannungsquelle einen Mikrocontroller zur Ansteuerung der Zusatzspannungsumschalteinrichtung aufweisen. Somit können die Umschaltung der Zusatzspannungsumschalteinrichtung und die Messwertaufnahme/-auswertung synchronisiert werden. Die entsprechenden Informationen stehen dann digital im Mikrocontroller zur Verfügung und können weiterverarbeitet werden. Dadurch kann der Schaltungsaufwand noch weiter verringert werden und eine digitale Auswertung/Verarbeitung der Information der erfindungsgemäßen Feuchtigkeitsdetektion ist möglich.

Im Rahmen der vorliegenden Erfindung wird auch ein Verfahren zur Feuchtigkeitsdetektion an einem Messspannungseingang einer Messvorrichtung mit zumindest einem Eingangskontakt für eine Eingangsspannung U_{E} von einem Messelement gemäß Anspruch 5 vorgeschlagen, wobei bei diesem Verfahren erfindungsgemäß eine Zusatzspannungsquelle und ein mit der Zusatzspannungsquelle verbundener Zusatzkontakt, welcher im Bereich des zumindest einen Eingangskontakts angeordnet wird, verwendet werden, wobei von der Zusatzspannungsquelle zumindest eine Zusatzspannung U_{Z} erzeugt wird und an den Zusatzkontakt angelegt wird, so dass bei Auftreten von Feuchtigkeit im Bereich des zumindest einen Eingangskontakts ein Nebenschluß zwischen dem zumindest einen Eingangskontakt und dem Zusatzkontakt hergestellt wird, so dass eine Größe der Eingangsspannung U_{E} bzw. in Folge auch der Messpannung U_{M} in Richtung einer Größe der zumindest einen Zusatzspannung U_{Z} gezogen wird. Die entsprechenden Vorgänge beim Ablauf des erfindungsgemäßen Verfahrens sind oben bereits im Zusammenhang mit der Vorstellung der erfindungsgemäßen Messvorrichtung beschrieben worden.

Bevorzugt kann bei dem erfindungsgemäßen Verfahren die Zusatzspannung U_{Z} mit einer Größe geliefert werden, welche außerhalb eines Bereichs einer Größe der Eingangsspannung U_{E} liegt.

Ferner kann bei dem erfindungsgemäßen Verfahren bevorzugt durch Umschalten zwischen zumindest einer ersten Zusatzspannung U_{Z1} und einer zweiten Zusatzspannung U_{Z2} eine noch weiter verbesserte Detektion von Feuchtigkeit erfolgen. Bevorzugt kann dabei als zweite Zusatzspannung U_{Z2} ein positives Spannungspotential V_{CC} eingesetzt werden.

Im Übrigen sei hinsichtlich des erfindungsgemäßen Verfahrens auf die entsprechenden Ausführungen im Zusammenhang mit der erfindungsgemäßen Vorrichtung verwiesen, wobei die erfindungsgemäße Vorrichtung zum Ausführen des erfindungsgemäßen Verfahrens entsprechend vorgesehen und ausgelegt ist.

Insgesamt ermöglicht die vorliegende Erfindung auf einfache und zuverlässige Art und Weise eine Feuchtigkeitsdetektion an einem Messspannungseingang einer Messvorrichtung.

Die Erfindung wird im Folgenden anhand von drei Ausführungsbeispielen mehr im Detail im Zusammenhang mit den beigefügten Zeichnungen beschrieben werden. In den Zeichnungen zeigen:
- Fig. 1: eine schematische Ansicht einer Messvorrichtung gemäß des Standes der Technik;
- Fig. 2: eine schematische Ansicht eines ersten Ausführungsbeispiels einer erfindungsgemäßen Messvorrichtung;
- Fig.3: eine schematische Ansicht eines zweiten Ausführungsbeispiels einer erfindungsgemäßen Messvorrichtung; und
- Fig. 4: eine schematische Ansicht eines zweiten Ausführungsbeispiels einer erfindungsgemäßen Messvorrichtung.

In der Fig. 1 ist schematisch eine Ansicht einer Messvorrichtung gemäß des Standes der Technik gezeigt, wie sie weiter oben bereits beschreiben wurde.

In der Fig. 2 ist schematisch ein erstes Ausführungsbeispiel einer erfindungsgemäßen Messvorrichtung dargestellt. In einem Anschlussbereich der erfindungsgemäßen Messvorrichtung ist ein Messspannungseingang 1 vorgesehen, an welchem eine Eingangsspannung U_{E} von einem Messelement (in dieser Figur und den übrigen Figuren nicht gezeigt) zugeführt ist. Der Messspannungseingang 1 weist zumindest einen Eingangskontakt 2 auf. Ferner ist ein Referenz-Eingangskontakt 7 vorgesehen, welcher als Referenzpotential auf Masse gelegt ist. Im Bereich des Eingangskontakts 2 ist ein Zusatzkontakt 4 erfindungsgemäß vorgesehen, welcher den Eingangskontakt 4 weitestgehend umgibt. Dieser Zusatzkontakt 4 ist mit einer Zusatzspannungsquelle 3 verbunden, welche eine Zusatzspannung U_{Z} liefert, und bildet so ein Zusatznetz. Eine Einheit zur Messsignalverarbeitung beinhaltet einen Impedanzwandler 8 mit einem Innenwiderstand R_{I}, wobei der Eingang des Impedanzwandlers mit dem (hochohmigen) Eingangskontakt 2 des Messspannungseingangs 1 verbunden ist. In weiteren Schaltungsteilen kann das vom Impedanzwandler 8 ausgegebene Messsignal mit einer Messspannung U_{M} in bekannter Art und Weise weiterverarbeitet werden.

Bei einem Auftreten von Feuchtigkeit im Bereich des Anschlussbereichs des Messspannungseingangs 1 treten ein erster Leckwiderstand R_{F1} zwischen dem Eingangskontakt 2 und dem Zusatzkontakt 4 und ein zweiter Leckwiderstand R_{F2} zwischen dem Eingangskontakt 2 und dem Referenz-Eingangskontakt 7 auf. Erfindungsgemäß wird über den ersten Leckwiderstand R_{F1} das Potential des Eingangskontakts 2 in Richtung des Potentials der Zusatzspannung U_{Z} der Zusatzspannungsquelle 3 gezogen. Liegt nun die Größe des Potentials der Zusatzspannung U_{Z} außerhalb des Bereichs der Eingangsspannung U_{E}, kann das Vorliegen von Feuchtigkeit im Anschlussbereich erfindungsgemäß einfach aufgrund des Herauslaufens der Eingangsspannung U_{E} aus dem normalen Bereich der Messspannungseingang-Eingangsspannung U_{E} festgestellt werden bzw. aufgrund des entsprechenden Herauslaufens der Messspannung UM aus dem normalen Bereich der Messspannung U_{M} festgestellt werden.

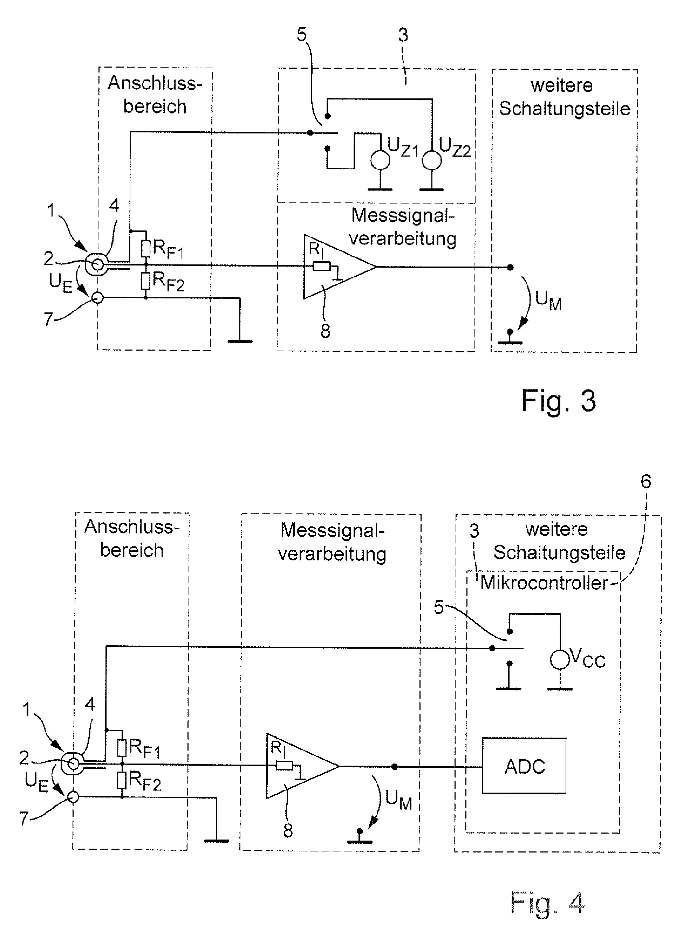

In der Fig. 3 ist schematisch ein zweites Ausführungsbeispiel einer erfindungsgemäßen Messvorrichtung dargestellt. Ebenso wie bei dem vorherigen Ausführungsbeispiel der Fig. 2 ist hier das Auftreten von Feuchtigkeit im Bereich des Anschlussbereichs der erfindungsgemäßen Messvorrichtung durch die entsprechenden Leckwiderstände, nämlich den ersten Leckwiderstand R_{F1} und den zweiten Leckwiderstand R_{F2}, dargestellt.

Hier erfolgt eine Detektion des fehlerbehafteten Auftretens von Feuchtigkeit durch Gestaltung der erfindungsgemäßen Zusatzspannungsquelle 3 mit einer Zusatzspannungsumschalteinrichtung 5, welche zwischen einer ersten Zusatzspannung U_{Z1} und einer zweiten Zusatzspannung U_{Z2} der Zusatzspannungsquelle 3 umschaltbar ist. Die übrigen Elemente gemäß Fig. 3 entsprechen den bereits in Fig. 2 gezeigten Elementen.

Gemäß der in Fig. 3 gezeigten Ausführungsform der erfindungsgemäßen Messvorrichtung kann also das dortige Zusatznetz aus Zusatzspannungsquelle 3 mit einer ersten Zusatzspannungsquelle für eine erste Zusatzspannung U_{Z1} und eine zweite Zusatzspannungsquelle für eine zweite Zusatzspannung U_{Z2}, der Zusatzspannungsumschalteinrichtung 5 und dem Zusatzkontakt 4 wechselweise zwischen zwei definierten Potentialen umschaltbar sein. Dringt nun Feuchtigkeit in den Anschlussbereich des Messspannungseingangs der erfindungsgemäßen Messvorrichtung mit dieser Gestaltung ein, werden das Zusatznetz und der hochohmige Eingangskontakt 2 über den ersten Leckwiderstand R_{F1} in Verbindung gelangen und das Eingangspotential wird in Richtung des Potentials der Zusatzspannung U_{Z} des Zusatznetzes gezogen, wobei aufgrund der Umschaltbarkeit der Zusatzspannung U_{Z} zwischen einer ersten Zusatzspannung U_{Z1} und einer zweiten Zusatzspannung U_{Z2} der Spannungsverlauf der Eingangsspannung U_{E} und/oder der Messspannung UM in Abhängigkeit der Schaltvorgänge bei Umschaltung von der ersten Zusatzspannung U_{Z1} auf die zweite Zusatzspannung U_{Z2} und zurück der Richtung der Spannungsänderung zwischen den beiden Zusatzspannungen folgen wird.

In der Fig. 4 ist schematisch ein drittes Ausführungsbeispiel einer erfindungsgemäßen Messvorrichtung dargestellt, welches im Wesentlichen dem zweiten Ausführungsbeispiel der Fig. 3 entspricht. Ebenso wie bei dem vorherigen, zweiten Ausführungsbeispiel der Fig. 3 ist in dieser Messvorrichtung eine Zusatzspannungsumschalteinrichtung 5 vorgesehen.

Diese schaltet zwischen einem Masse-Referenzpotential (entspricht z.B. der ersten Zusatzspannung U_{Z1} im zweiten Ausführungsbeispiel) und einem bevorzugt positiven Spannungspotential V_{CC} (entspricht z.B. der zweiten Zusatzspannung U_{Z2} im zweiten Ausführungsbeispiel) erfindungsgemäß um, wobei die Steuerung dabei über einen Mikrocontroller 6 erfolgt. Die Signalverarbeitung erfolgt über einen Analog-Digital-Wandler (ADC).

Hinsichtlich eines möglichen Platinenlayouts ist zu beachten, dass das Zusatznetz insbesondere in einem Bereich, in welchem mit Feuchtigkeitsansammlung zu rechnen sein wird, also z.B. im Anschlussbereich, nicht mit Lötstopplack bedeckt sein soll, da dann kein Kontakt insbesondere des Zusatzkontaktes mit der Feuchtigkeit auftreten könnte.

### Bezugszeichenliste

- 1: Messspannungseingang
- 2: Eingangskontakt
- 3: Zusatzspannungsquelle
- 4: Zusatzkontakt
- 5: Zusatzspannungsumschalteinrichtung
- 6: Mikrocontroller
- 7: Referenz-Eingangskontakt
- 8: Impedanzwandler

- U_{E}: Eingangsspannung
- U_{Z}: Zusatzspannung
- U_{Z1}: erste Zusatzspannung
- U_{Z2}: zweite Zusatzspannung
- UM: Messspannung
- V_{CC}: positives Spannungspotential

- R_{F}: Leckwiderstand
- R_{F1}: erster Leckwiderstand
- R_{F2}: zweiter Leckwiderstand
- R_{I}: Eingangswiderstand

## Patentansprüche

1. Messvorrichtung mit einem Messspannungseingang (1) mit zumindest einem Eingangskontakt (2) für eine Eingangsspannung (U_{E}) von einem Messelement,
**dadurch gekennzeichnet, dass**
die Messvorrichtung eine Zusatzspannungsquelle (3) aufweist, welche zumindest eine Zusatzspannung (U_{Z}) liefert, verbunden mit einem Zusatzkontakt (4), angeordnet im Bereich des zumindest einen Eingangskontakts (2).

2. Messvorrichtung nach Anspruch 1, **dadurch gekennzeichnet, dass** die Zusatzspannungsquelle (3) so vorgesehen ist, dass sie zumindest eine Zusatzspannung (U_{Z}) einer Größe liefert, welche außerhalb eines Bereichs einer Größe der Eingangsspannung (U_{E}) liegt.

3. Messvorrichtung nach Anspruch 1 oder 2, **dadurch gekennzeichnet, dass** die Zusatzspannungsquelle (3) so vorgesehen ist, dass sie zumindest eine erste Zusatzspannung (U_{Z1}) und einer zweiten Zusatzspannung (U_{Z2}) liefert und eine Zusatzspannungsumschalteinrichtung (5) aufweist, mit welcher zuwischen der ersten Zusatzspannung (U_{Z1}) und der zweiten Zusatzspannung (U_{Z2}) umschaltbar ist, wobei die zweiten Zusatzspannung (U_{Z2}) bevorzugt ein positives Spannungspotential (V_{CC}) ist.

4. Messvorrichtung nach Anspruch 3, **dadurch gekennzeichnet, dass** die Zusatzspannungsquelle (3) einen Mikrocontroller (6) zur Ansteuerung der Zusatzspannungsumschalteinrichtung (5) aufweist.

5. Verfahren zur Feuchtigkeitsdetektion an einem Messspannungseingang einer Messvorrichtung mit zumindest einem Eingangskontakt für eine Eingangsspannung (U_{E}) von einem Messelement,
**gekennzeichnet durch**
Verwenden einer Zusatzspannungsquelle und eines mit der Zusatzspannungsquelle verbunden Zusatzkontakts, welcher im Bereich des zumindest einen Eingangskontakts angeordnet wird, wobei von der Zusatzspannungsquelle zumindest eine Zusatzspannung (U_{Z}) erzeugt wird und an den Zusatzkontakt angelegt wird, so dass bei Auftreten von Feuchtigkeit im Bereich des zumindest einen Eingangskontakts ein Nebenschluss zwischen dem zumindest einen Eingangskontakt und dem Zusatzkontakt hergestellt wird, so dass eine Größe der Eingangsspannung (U_{E}) in Richtung einer Größe der zumindest einen Zusatzspannung (U_{Z}) gezogen wird.

6. Verfahren nach Anspruch 5, **gekennzeichnet durch** Liefern einer Zusatzspannung (U_{Z}) mit einer Größe, welche außerhalb eines Bereichs einer Größe der Eingangsspannung (U_{E}) liegt.

7. Verfahren nach Anspruch 5 oder 6, **gekennzeichnet durch** Umschalten zuwischen zumindest eine erste Zusatzspannung (U_{Z1}) und einer zweiten Zusatzspannung (U_{Z2}), wobei als zweite Zusatzspannung (U_{Z2}) bevorzugt ein positives Spannungspotential (V_{CC}) eingesetzt wird.
